(19) Europäisches Patentamt
European Patent Office
Office européen des brevets

(11) Veröffentlichungsnummer : **0 134 828**
**B1**

(12) # EUROPÄISCHE PATENTSCHRIFT

(45) Veröffentlichungstag der Patentschrift : **05.04.89**

(51) Int. Cl.[4] : **C 07 D213/64**, C 07 D213/79

(21) Anmeldenummer : **83108936.2**

(22) Anmeldetag : **09.09.83**

(54) **Verfahren zur Herstellung von 2-Hydroxypyridinen aus 2-Pyridincarbonsäure-N-Oxiden.**

(43) Veröffentlichungstag der Anmeldung : **27.03.85 Patentblatt 85/13**

(45) Bekanntmachung des Hinweises auf die Patenterteilung : **05.04.89 Patentblatt 89/14**

(84) Benannte Vertragsstaaten : **AT BE CH DE FR GB IT LI NL SE**

(56) Entgegenhaltungen :
**EP--A-- 0 084 118**
**JOURNAL OF THE CHEMICAL SOCIETY, Dezember 1961, Seiten 5216-5223, B.M. BAIN u.a.: "The reaction of nicotinic acid 1-oxide and 3-picoline 1-oxide with acetic anhydride"**
**BULLETIN OF THE CHEMICAL SOCIETY OF JAPAN, Band 42, Nr. 11, November 1969, Seiten 3350-3352, Y. MURAKAMI u.a.: "Reaction of 6-methylpicolinic acid N-oxide with acetic anhydride"**

(73) Patentinhaber : **LONZA AG**
**Gampel/Wallis (CH)**

(72) Erfinder : **Quarroz, Daniel, Dr.**
**Balfrinstrasse 21**
**Visp (Kanton Wallis) (CH)**

(74) Vertreter : **Weinhold, Peter, Dr. et al**
**Patentanwälte Dr. V. Schmied-Kowarzik Dipl.-Ing. G. Dannenberg Dr. P. Weinhold Dr. D. Gudel Dipl.-Ing. S. Schubert Dr. P. Barz Siegfriedstrasse 8**
**D-8000 München 40 (DE)**

**EP 0 134 828 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von 2-Hydroxypyridinen, insbesondere von 2-Hydroxypyridincarbonsäure und 2-Hydroxyalkylpyridin.

Es ist bekannt, dass 2-Hydroxypyridine durch Diazotieren der entsprechenden Aminopyridine und anschliessenden Behandlung mit Alkalilauge oder aus Pyridinsulfonsäuren oder Halogenpyridinen durch Austausch mit Alkali, gegebenenfalls mittels Metallkatalysatoren, wie Kupfer, erhalten werden können. Ferner ist in der Literatur beschrieben, das sich Pyridin-N-Oxid mit Essigsäureanhydrid zu 2-Acetoxypyridin umsetzt, das durch Hydrolyse in 2-Hydroxypyridin bzw. 2-Pyridon umgewandelt werden kann. Auf der anderen Seite liefert die Reaktion von Picolinsäure-N-Oxid mit Essigsäureanhydrid nur in geringen Mengen 2-Hydroxypyridin. Als Hauptprodukt entsteht Pyridin-N-Oxid (J. Chem. Soc. 1961, 5216). Wenn man die gleiche Reaktion mit Isocinchomeronsäure durchführt, erhält man in analoger Weise nur Spuren 6-Hydroxynikotinsäure und Nikotinsäure-N-Oxid als Hauptprodukt. Eine Ausnahme stellt die Reaktion von 6-Methylpicolinsäure mit Essigsäureanhydrid dar, die in guter Ausbeute 2-Hydroxy-6-methylpyridin liefert (Bull. Chem. Soc. Japan 42, 3350, 1969).

Ziel der vorliegenden Erfindung ist es, 2-Hydroxypyridincarbonsäuren und 2-Hydroxyalkylpyridine in hohen Ausbeuten, insbesondere Raum-Zeit-Ausbeuten, herzustellen.

Gegenstand der Erfindung ist ein Verfahren zur Herstellung von 2-Hydroxypyridinen der allgemeinen Formel

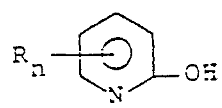

worin R = H, eine —COOH-Gruppe oder eine Alkylgruppe mit $C_1$-$C_8$ oder eine Arylgruppe und n die Zahl 1,2,3 oder 4 bedeutet, mit der Maßgabe, daß in 6-Stellung keine Methylgruppe auftritt, das dadurch gekennzeichnet ist, daß man ein 2-Pyridincarbonsäure-N-Oxid der allgemeinen Formel

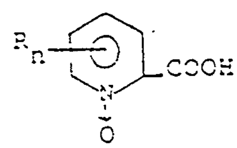

wobei R und n die oben genannten Bedeutungen haben, mit einem niederen aliphatischen Carbonsäureanhydrid in Gegenwart eines tertiären Amins umsetzt und das Umsetzungsprodukt hydrolisiert.

Als niedere aliphatische Anhydride werden insbesondere Essigsäureanhydrid und Propionsäureanhydrid, vorzugsweise Essigsäureanhydrid, eingesetzt.

Das Carbonsäureanhydrid wird, bezogen auf das eingesetzte Pyridincarbonsäure-N-Oxid in 1- bis 10-fachem molaren Überschuß vorgelegt und kann auch als Lösungsmittel dienen.

Nach diesem Verfahren erhält man überraschenderweise 2-Hydroxypyridincarbonsäuren in hohen Ausbeuten, wobei die Reaktion bereits bei relativ tiefen Temperaturen mit großer Geschwindigkeit abläuft.

Das erfindungsgemäße Verfahren kann auch in Anwesenheit von Lösungsmitteln, wie z. B. Toluol, Petroläther, $CCl_4$, Essigsäureäthylester, Essigsäure, Acetonitril usw., durchgeführt werden.

Als Katalysator können neben Triäthylamin auch andere aliphatische oder aliphatisch/aromatische tertiäre Amine, Pyridin oder DMF verwendet werden. Vorzugsweise werden als aliphatische Amine Tri-$C_1$-$C_4$-alkylamin, insbesondere Triäthylamin eingesetzt und kommt ins Verhältnis tertiäres Amin zu Pyridincarbonsäure-N-Oxid zwischen 1 zu 1 bis 20 zu 1, vorzugsweise bei 5 zu 1 bis 2 zu 1, zur Verwendung.

Als Ausgangsprodukte kommen neben dem 2-Pyridincarbonsäure-N-Oxid auch die entsprechenden Pyridindicarbonsäure-N-Oxide, wie

- Pyridindicarbonsäure-(2,3)-N-Oxid (Chinolinsäure)
- Pyridindicarbonsäure-(2,4)-N-Oxid (Lutidinsäure)
- Pyridindicarbonsäure-(2,5)-N-Oxid (Isocinchomeronsäure)
- Pyridindicarbonsäure-(2,6)-N-Oxid (Dipicolinsäure)

die entsprechenden Pyridintricarbonsäure-N-Oxide, wie

- Pyridintricarbonsäure-(2,3,4)-N-Oxid (α-Carbocinchomeronsäure)
- Pyridintricarbonsäure-(2,4,5)-N-Oxid (Berberonsäure)
- Pyridintricarbonsäure-(2,4,6)-N-Oxid (Trimesitinsäure)

und Pyridinpentacarbonsäure-N-Oxid in Frage.

Die angeführten Pyridincarbonsäure-N-Oxide können ausserdem 1- bis 4-fach mit Alkyl der Kettenlänge $C_1$...$C_8$, d. h. Methyl, Äthyl, Propyl, Butyl, Pentyl, Hexyl, Heptyl, Octyl oder mit Aryl, vorzugsweise

Phenyl, kernsubstituiert sein. Die Umsetzungsreaktion wird bei einer Temperatur von 0 bis 50 °C vorzugsweise bei 20 bis 30 °C, durchgeführt.

Die erfindungsgemäß hergestellten 2-Hydroxypyridine, insbesondere die 6-Hydroxynikotinsäure, stellen wertvolle Zwischenprodukte dar, z. B. für die Herstellung von Herbiciden, wie 5-Trifluoromethyl-2-pyridinon (CA 94, 121, 342n), oder zur Herstellung von 6-Chlornikotinsäure, die ihrerseits wieder als Zwischenprodukt zur Herstellung von 2-Amino-5-pyridinsäureester und -amid dient (CA 90, 174, 689c, CA 88, 141 704 m). Ein Folgeprodukt des aus 2-Hydroxypyridin herstellbaren 2-Chlorpyridins ist das Omadin (Pyridin-2-thiol-1-oxid), ein Mittel gegen Kopfhautschuppen.

### Beispiel 1

Herstellung von 6-Hydroxynikotinsäure (6-OHNS) aus Isocinchomeronsäure-N-Oxid (ICSO).

200 g Essigsäureanhydrid (1,96 Mol) und 50 g Triäthylamin (0,5 Mol) wurden in einem Kolben vorgelegt und 36 g ICSO (0,197 Mol) bei Raumtemperatur portionsweise so zudosiert, dass die Reaktionstemperatur 30 °C nicht überschritt. Nach beendeter Zugabe liess man noch ca. 1 Stunde bei 30 °C nachreagieren, bis kein $CO_2$ mehr entwich. Die resultierende braunschwarze Lösung wurde am Rotavapor (30 Torr, 60 °C) eingeengt und der dickflüssige Rückstand durch Zusatz von 20 %iger KOH (End-pH ~ 12), bei 80 °C, 1/4 Stunde verseift.

Zur Entfernung des Triäthylamins wurde das flüssige Reaktionsgemisch mit $CH_2Cl_2$ extrahiert und anschliessend mit konzentrierter HCl angesäuert (pH 1). Der hierbei angefallene Niederschlag wurde abgenutscht, mit $H_2O$ gewaschen und bei 45 °C, 20 Torr, getrocknet.

Ausbeute : 23 g (titrimerisch bestimmter Gehalt 96,5 %), entsprechend 81 % der Theorie.

### Beispiel 2

Herstellung von 2-Hydroxynikotinsäure (2-OHNS) aus Chinolinsäure-N-Oxid (CSO).

100 g Essigsäureanhydrid (0,98 Mol) und 25 g Triäthylamin (0,25 Mol) wurden vorgelegt und auf 40 °C erwärmt, um eine lebhafte Gasentwicklung während der portionsweisen Zugabe von 18 g CSO (0,098 Mol) zu gewährleisten.

Die Aufarbeitung des Reaktionsgemisches erfolgte analog Beispiel 1.

Ausbeute : 5,6 g nach NMR reine 2-OHNS (ca. 41 % der Theorie).

### Beispiel 3

Herstellung von 2-Hydroxypyridin aus 2-Pyridincarbonsäure-N-Oxid (Picolinsäure-N-Oxid ; PSO).

200 g Essigsäureanhydrid (1,96 Mol) und 40 g Triäthylamin (0,4 Mol) wurden in einem Kolben vorgelegt und 36 g PSO (0,26 Mol) zudosiert. Die Rekationstemperatur wurde während der Zugabe des PSO zwischen 20 und 30 °C gehalten.

Nach beendeter Reaktion wurde die Flüssigkeit auf dem Rotavapor eingedampft und der Rückstand mit Wasser aufgenommen. Dieser wässrigen Lösung wurde langsam 30 %ige NaOH zugefügt, bis kein weiterer Niederschlag entstand. Das so erhaltene Na-Salz des 2-Hydroxypyridins wurde abgenutscht, mit 30 %iger NaOH gewaschen, aus 95 % Alkohol umkristallisiert und bei 45 °C, 20 Torr, getrocknet.

Ausbeute : 30,5 g nach H-NMR reines 2-Hydroxypyridin-Na-Salz. Da das erhaltene 2-Hydroxypyridin-Na-Salz noch ca. 3 % $H_2O$ enthielt, ergab dies ca. 95 % der Theorie.

### Beispiel 4

Herstellung von 6-Hydroxynikotinsäure.

20,4 g Essigsäureanhydrid (0,2 Mol), 20,2 g $Et_3N$ (0,2 Mol) und 50 ml $CCl_4$ wurden in einen Kolben vorgelegt. 18,3 g Isocinchomeronsäure-N-Oxid (0,1 Mol) wurden portionsweise dieser Lösung zugegeben. Die Temperatur stieg bis 50 °C. Nach Ende der Reaktion wurden die Lösungsmittel abdestilliert. Zu dem dickflüssigen Rückstand wurden ca. 100 ml KOH 20 % zugetropft und das Acetat wurde während 15 Minuten bei 80 °C verseift. Nach Entfernung des $Et_3N$ durch Extraktion wurde die wässrige Lösung durch HCl konz. angesäuert. Der Niederschlag wurde abgenutscht, mit Wasser gewaschen und unter Vakuum getrocknet. Man bekam 111,0 g 6-Hydroxynikotinsäure, die nach NMR rein war. Die Rohausbeute lag bei 79 %.

### Beispiel 5

Herstellung von 6-Hydroxynikotinsäure.

Wurde wie in Beispiel 1 vorgegangen, aber Propionsäureanhydrid anstelle von Essigsäureanhydrid verwendet, betrug die Ausbeute an 6-Hydroxynikotinsäure 78 %.

### Beispiel 6

Herstellung von 6-Hydroxynikotinsäure.

Wurde wie in Beispiel 1 vorgegangen, aber Tributylamin anstelle von $Et_3N$ verwendet, betrug die Ausbeute an 6-Hydroxynikotinsäure 75 %.

Beispiel 7

Herstellung von 6-Hydroxynikotinsäure.

Wurde wie in Beispiel 4 vorgegangen, aber Petroläther anstelle von $CCl_4$ verwendet, betrug die Ausbeute an 6-Hydroxynikotinsäure 67 %.

Beispiel 8

Herstellung von 6-Hydroxypicolinsäure.

Essigsäureanhydrid (0,5 Mol) und $Et_3N$ (0,15 Mol) wurden in einen Kolben vorgelegt. Dipicolinsäure-N-Oxid (0,05 Mol) wurde portionsweise in diese Lösung gegeben ; die Temperatur betrug 35 °C. Nach Ende der Zugabe wurde die Lösung am Rotavapor eingedampft. Zum Rückstand wurden 80 ml KOH 20 % gegeben und 15 Minuten bei 80 °C gehalten. Nach Extraktion des $Et_3N$ mit $CH_2Cl_2$ wurde die wässrige Phase mit HCl konz. bis pH 2 angesäuert. Die Kristalle wurden abgenutscht, mit Wasser gewaschen und getrocknet.

Man erhielt 4,2 g 6-Hydroxypicolinsäure, die nach NMR rein war. Die Rohausbeute lag bei 60 %.

**Patentansprüche**

1. Verfahren zur Herstellung von 2-Hydroxypyridinen der allgemeinen Formel

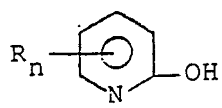

worin R = H, eine —COOH-Gruppe oder eine Alkylgruppe mit $C_1$-$C_8$ oder eine Arylgruppe und n die Zahl 1, 2, 3 oder 4 bedeutet, mit der Maßgabe, daß in 6-Stellung keine Methylgruppe auftritt, dadurch gekennzeichnet, daß man ein 2-Pyridincarbonsäure-N-Oxid der allgemeinen Formel

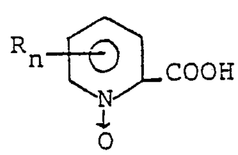

wobei R und n die oben genannten Bedeutungen haben, mit einem niederen aliphatischen Carbonsäureanhydrid in Gegenwart eines tertiären Amins umsetzt und das Umsetzungsprodukt hydrolisiert.

2. Verfahren gemäss Patentanspruch 1, dadurch gekennzeichnet, dass man als Carbonsäureanhydrid Essigsäureanhydrid verwendet.

3. Verfahren gemäss Patentansprüchen 1 und 2, dadurch gekennzeichnet, dass als tertiäres Amin Triäthylamin verwendet wird.

4. Verfahren gemäss Patentansprüchen 1 bis 3, dadurch gekennzeichnet, dass das Verhältnis von tertiärem Amin zu 2-Pyridincarbonsäure-N-Oxid zwischen 1 zu 1 bis 20 zu 1, vorzugsweise bei 5 zu 1 bis 2 zu 1, liegt.

5. Verfahren gemäss Patentansprüchen 1 bis 4, dadurch gekennzeichnet, dass man die Reaktion bei einer Temperatur von 0 bis 50 °C, vorzugsweise bei 20 bis 30 °C, durchführt.

6. Verfahren gemäss Patentansprüchen 1 bis 5, dadurch gekennzeichnet, dass man die Reaktion in Anwesenheit eines Lösungsmittels durchführt.

**Claims**

1. Method for the preparation of 2-hydroxypyridines of the general formula

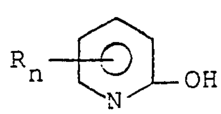

wherein R is H, a —COOH-group or an alkyl group with $C_1$-$C_8$ or an aryl group, and n is 1, 2, 3 or 4, with

the proviso that there is no methyl group in the 6-position, characterized in that a 2-pyridine carboxylic acid-N-oxide of the general formula

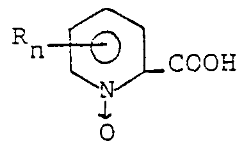

wherein R and n have the above mentioned meanings, is reacted with a low aliphatic carboxylic acid anhydride in the presence of a tertiary amine and the reaction product is hydrolyzed.

2. Method according to patent claim 1, characterized in that acetic anhydride is used as carboxylic acid anhydride.

3. Method according to patent claims 1 and 2, characterized in that triethylamine is used as tertiary amine.

4. Method according to patent claims 1 to 3, characterized in that the ratio of tertiary amine to 2-pyridine carboxylic acid-N-oxide is between 1 : 1 to 20 : 1, preferably at 5 : 1 to 2 : 1.

5. Method according to patent claims 1 to 4, characterized in that the reaction is effected at a temperature of 0 to 50 °C, preferably at 20 to 30 °C.

6. Method according to patent claims 1 to 5, characterized in that the reaction is effected in the presence of a solvent.

**Revendications**

1. Procédé pour la préparation de 2-hydroxypyridines de formule générale

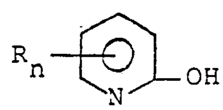

dans laquelle R=H, un groupe —COOH ou un groupe alcoyle en $C_1$-$C_8$ ou un groupe aryle et n représente le nombre 1, 2, 3 ou 4, étant entendu qu'il n'y a pas de groupe méthyle en position 6, caractérisé en ce qu'on fait réagir un acide 2-pyridinecarboxylique-N-oxyde de formule générale

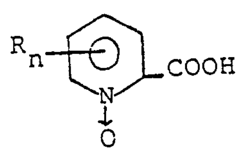

dans laquelle R et n ont les significations mentionnées plus haut, avec un anhydride d'acide carboxylique aliphatique inférieur, en présence d'une amine tertiaire et en ce qu'on hydrolyse le produit de réaction.

2. Procédé selon la revendication 1, caractérisé en ce qu'on utilise, en tant qu'anhydride d'acide carboxylique, l'anhydride acétique.

3. Procédé selon les revendications 1 et 2, caractérisé en ce qu'on utilise comme amine tertiaire, la triéthylamine.

4. Procédé selon les revendications 1 à 3, caractérisé en ce que le rapport de l'amine tertiaire à l'acide 2-pyridinecarboxylique-N-oxyde se situe entre 1 à 1 jusqu'à 20 à 1 et est de préférence de 5 à 1 jusqu'à 2 à 1.

5. Procédé selon les revendications 1 à 4, caractérisé en ce qu'on effectue la réaction à une température de 0 à 50 °C, de préférence à une température de 20 à 30 °C.

6. Procédé selon les revendications 1 à 5, caractérisé en ce que la réaction est effectuée en présence d'un solvant.